# EUROPEAN PATENT APPLICATION

(11) **EP 4 806 428 A1**
(43) Date of publication of application: **16.09.2026**
(21) Application number: 26163487.7
(22) Date of filing: 10.03.2026
(51) Int. Cl.: A61K 9/16, A61K 9/20, A61K 31/47

(54) **PHARMACEUTICAL FORMULATIONS OF IVACAFTOR**

(30) Priority: 13.03.2025 US 202563771169 P
(71) Applicant: Boston Biopharma, Inc., Alpharetta, GA 30009 (US)
(72) Inventor: HASHEM, Ahmad, Alpharetta, 30009 (US); CAUCHI, Maria, Alpharetta, 30009 (US)
(74) Representative: Abel & Imray LLP

(57) **Abstract**

The present invention relates to a solid oral pharmaceutical composition comprising ivacaftor or a pharmaceutically acceptable salt thereof, as well as methods for making and using such compositions. Compositions comprised Micronized ivacaftor having D₉₀ not more than 25 µm and D₅₀ not more than 15 µm for tablets and for Granules/mini tablets D₉₀ not more than 5 µm.

## Description

### FIELD OF INVENTION:

The present invention relates to a novel composition that comprises CFTR modulators as well as methods for preparing it, wherein said composition is used for treating diseases and conditions that are beneficially treated by administering CFTR (cystic fibrosis transmembrane conductance regulator) modulators, i.e., cystic fibrosis (CF).

### BACKGROUND OF THE INVENTION:

Ivacaftor is chemically known as N-(2,4-ditertbutyl-5-hydroxyphenyl)-1,4-dihydro-4-oxoquinoline-3-carboxamide having a structural formula as represented by formula I. 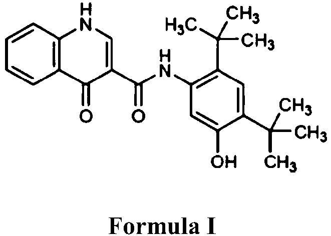 It is a potentiator of the CFTR (cystic fibrosis transmembrane conductance regulator) protein and has demonstrated clinical activity against cystic fibrosis.

The FDA approved Ivacaftor in 2012 for the treatment of cystic fibrosis in patients with the G55 1D-CFTR gene mutation. Ivacaftor was granted fast track and orphan drug designations by the FDA in 2006 and 2007, respectively, and is marketed under the brand name Kalydeco^{®}. Ivacaftor is also approved in combination with VX-809 (also known as lumacaftor, a CFTR corrector) for the oral treatment of cystic fibrosis patients carrying the more common AF508-CFTR mutation; the combination is marketed under the brand name Orkambi^{®}. Ivacaftor is also approved in combination with Tezacaftor for the oral treatment of cystic fibrosis (CF) in patients age 6 years and older who are homozygous for the F508del mutation; the combination is marketed under the brand name SYMDEKO^{®}. Ivacaftor is also approved in combination with Elexacaftor and Tezacaftor for the oral treatment of cystic fibrosis (CF) in patients aged 2 years and older who have at least one F508del mutation in the cystic fibrosis transmembrane conductance regulator (CFTR) gene; the combination is marketed under the brand name TRIKAFTA^{®}.

Vertex markets ivacaftor under the brand name Kalydeco^{®}, and it is disclosed in US 7,495,103, which includes modulators of ATP-binding cassette transporters such as ivacaftor as well as methods for treating CFTR transporter-mediated diseases using such modulators.

U.S. Patent No. 7,553,855 describes a pharmaceutical composition that includes Ivacaftor, PEG 400, and PVP K30. U.S. Patent No. 8,410,274 describes pharmaceutical compositions that include ivacaftor, as well as an amorphous solid dispersion containing 50 wt% ivacaftor and 50 wt% polymer.

U.S. Patent No. 10,646,481 discloses a pharmaceutical composition comprising a solid dispersion of amorphous or substantially amorphous Ivacaftor, HPMCAS and SLS, as well as methods of manufacture.

U.S. Patent No. 11,752,106 discloses a pharmaceutical composition comprising a solid dispersion of amorphous or substantially amorphous Ivacaftor, HPMCAS, SLS, as well as other pharmaceutically acceptable excipients such as a sweetener, disintegrant, glidant, lubricant, etc.

U.S. Patent No. 8,163,772 discloses solid forms of ivacaftor in the form of co-forms with 2-methyl butyric acid, propylene glycol, PEG400.KOAc, lactic acid, isobutyric acid, propionic acid, ethanol, 2-propanol, water, besylate, hemibesylate, and besylate monohydrate.

U.S. Patent Publication No. 2015/0010628 discloses a pharmaceutical composition comprising a solid dispersion of amorphous or substantially amorphous ivacaftor, a filler, a sweetener, a disintegrant, a glidant, and a lubricant, and optionally a wetting agent.

U.S. Patent Publication No. 2024/0100183 discloses a pharmaceutical composition comprising particles, which comprises one or more CFTR modulators, wherein the particles have a diameter of about 1 µm to about 10 µm. with Cyclodextrin and Non- Ionic surfactant.

The PCT publication No. WO 2017/187336 describes a complex formulation of Ivacaftor or its salts or derivatives, complexation agents, and pharmaceutically acceptable excipients, with particle sizes ranging from 10 nm to 600 nm.

U.S. Patent No. 8,471,029 discloses crystalline form C of N-[2,4-bis(l,l -dimethyl ethyl)-5- hydroxyphenyl]-1,4-dihydro-4-oxoquinoline-3-carboxamide.

US Patent No. 8,674,108 discloses crystalline solvates of ivacaftor, which are designated as Form D, Form E, Form F, Form G, Form H, Form I, Form J, Form K, Form L, Form M, Form N, Form O, Form P, Form Q, Form R, Form S, Form T, Form W, and hydrate B, and their preparation.

The common use of solid dispersions in pharmaceutical delivery systems is to increase the solubility of very poorly soluble active ingredients and to stabilize the amorphous form of the drug substance after it has been molecularly dispersed in the polymeric matrix. There are various methods for producing solid dispersions. Hot-melt extrusion and spray-drying are the most common technologies for producing this type of delivery system.

Hot-melt extrusion is a manufacturing process that involves heating polymeric materials above their glass-transition temperature and mechanically mixing them with active compounds to molecularly disperse the drug substance into the polymeric net. Hot-melt extrusion equipment is expensive. The main disadvantages of this technology are: i) active compounds should not degrade at high temperatures; ii) the melting point of the active substance should be low enough to be dispersed molecularly; and iii) glass-transition temperatures of polymers must be low enough to allow for a feasible manufacturing process while remaining sufficiently high to stabilize the solid dispersion.

Spray-drying involves two major steps: i) complete dissolution of the drug substance and a dilution material (e.g., polymer) in a solvent or mixture of solvents; and ii) rapid spray-drying of the previous solution to collect the solid material. Spray-drying technology demonstrates numerous inconveniences: The use of organic solvents to dissolve the active ingredient is typically required because the latter has very poor solubility in aqueous media; however, dissolving the drug substance and excipients in a solvent may result in chemical reactions, and the resulting product after spray-drying usually has small particle size and very low bulk density, resulting in poor flow. This particular feature of the spray-dried material can be transferred to the final blend properties, making the tableting step extremely difficult. The main disadvantage of this technology is a time-consuming process, which is conditioned by the amount of solvent required to dissolve all of the components and the maximum spraying speed to have a feasible process.

However, the current invention focuses on novel CFTR modulator compositions and pharmaceutically acceptable salts thereof that have high solubility and can be easily formulated into a formulation with a desirable dissolving profile.

The current inventors surprisingly found pharmaceutical formulations comprising micronized Ivacaftor and a process for preparing them that are more commercially viable, less difficult, cost-effective and easier to commercialize pharmaceutical formulations of CFTR modulators, as well as manufacturing procedures for them. One such approach results in a composition that has the desired dissolving profile. These compositions exhibited the expected dissolution profile and bioavailability, which were also shown to be equivalent to commercially available Ivacaftor formulations, such as Kalydeco^{®}.

Furthermore, the current marketed product includes solid dispersion methods, which includes amorphous or substantially amorphous form of Ivacaftor. However, due to low solubility and bioavailability of the marketed KALYDECO^{®} tablet, it is manufactured in two stages: spray dispersion and tableting. As a result, a novel formulation containing micronized Ivacaftor was produced to ensure greater solubility, and the composition made using the direct compression or dry-granulation method is more economically viable, less challenging, cost-effective, and easier to commercialize pharmaceutical formulations of CFTR modulators.

### SUMMARY OF THE INVENTION:

In one aspect, the present invention provides novel CFTR modulator formulations as well as a method for preparing them.

In another aspect, the present invention provides a novel composition comprising at least one CFTR modulator and one or more pharmaceutically acceptable excipients, which is then formed into a suitable dosage form with one or more pharmaceutically acceptable excipients.

In another aspect, a novel composition of the present invention can also be described as solid, oral pharmaceutical compositions comprising a therapeutic amount of one or more CFTR modulators, which include CFTR potentiators and CFTR correctors, and at least one pharmaceutically acceptable excipient, wherein the disclosed composition of the invention can be processed into a tablet, mini-tablets, capsule form, granules, powders, pellets, or unit dose packets.

In yet another aspect, the novel compositions of the present invention can also be described as solid, oral pharmaceutical compositions comprising a therapeutic amount of one or more CFTR modulators and at least one pharmaceutically acceptable excipient, wherein said composition is substantially free of a solid dispersion technology and compressed into core tablets or mini-tablets with one or more excipients and optionally coated with a film coating.

In one aspect, at least one of the CFTR modulators is a CFTR potentiator. In addition, the CFTR modulator includes ivacaftor, FDL176, FDL169, GLPG2451, GLPG1837, QBW251, PTI-808, quercetin, and genistein. At least one of the CFTR modulators functions as a CFTR corrector. In addition, the CFTR corrector includes lumacaftor, Corr-4a, VRT-325, tezacaftor, elexacaftor, cavosonstat, FDL169, VX-152, VX-440, VX-445, or VX-659. Furthermore, the composition contains a CFTR potentiator and a CFTR corrector.

In one aspect, the composition of the present invention includes Ivacaftor, its salts, or derivatives thereof. In another way, the composition includes Ivacaftor and lumacaftor. In addition, the composition includes Ivacaftor and tezacaftor. In addition, composition includes Ivacaftor, tezacaftor, and elexacaftor.

In one aspect, the present invention provides a solid oral composition, which includes particles/granules comprising CFTR potentiator and one or more pharmaceutical acceptable excipients. The CFTR potentiator is Ivacaftor or its salts or derivatives, wherein Ivacaftor is micronized and has D₉₀ not more than 50 µm and D₅₀ not more than 25 µm. The micronized ivacaftor preferably has: (a) D₉₀ of not more than 25 µm; and D₅₀ of not more than 15 µm. Micronized ivacaftor preferably has D₉₀ not more than 25 µm, preferably between 25 µm and 15 µm, and D₅₀ not more than 15 µm, preferably between 5 µm and 15 µm, e.g. for tablets. Micronized ivacaftor preferably has D₉₀ not more than 5 µm for granules/mini tablets.

In one aspect, the present invention provides a novel pharmaceutical composition comprising Ivacaftor or a pharmaceutically acceptable salt thereof and one or more pharmaceutically acceptable excipients, wherein said composition comprises an intra-granular portion and an extra-granular portion.

In another aspect, the present invention provides a novel pharmaceutical composition comprising Ivacaftor or a pharmaceutically acceptable salt thereof and one or more pharmaceutically acceptable excipients, wherein said composition comprises an intra-granular portion and an extra-granular portion, wherein intra-granular portion comprises one or more excipients such as diluents, binders, disintegrants, surfactants etc. and wherein extra-granular portion comprising one or more excipients such as Lubricants, disintegrants, glidants etc.

In another aspect, the present invention provides a novel pharmaceutical composition comprising Micronized Ivacaftor and one or more pharmaceutically acceptable excipients, wherein said composition comprises an intra-granular portion and an extra-granular portion, wherein intra-granular portion comprises one or more diluents, one or more disintegrants, one or more surfactants and optionally one or more other excipients, and wherein extra-granular portion comprising one or more lubricants and optionally one or more other excipients.

In one aspect, the present invention provides a composition comprising at least one CFTR potentiator, i.e., Ivacaftor, and at least one filler and binder and made it into a suitable dosage form such as mini-tablets and tablets and optionally coated with film coating.

In another aspect, the present invention provides a novel solid oral composition, which includes a particle/granule containing a therapeutic amount of a CFTR potentiator, i.e., Ivacaftor, as well as at least one filler and binder, and which can be compressed into mini-tablets or core tablets and optionally coated with a film.

In another aspect, the present invention provides an immediate-release composition comprising Ivacaftor or its salts or derivatives, and at least one binder and at least one disintegrant, wherein said composition is made by using any of the granulation technologies, such as wet granulation and dry granulation, or the direct compression method, wherein said composition can be processed into a mini-tablet, tablet, or solution, filled into capsules.

In another aspect, the present invention provides an immediate-release solid oral composition comprising one or more therapeutic agents: a) at least one CFTR potentiator and b) at least one CFTR corrector and one or more pharmaceutically acceptable excipients, wherein said composition is made using any one of the granulation technology or direct compression method and made into a suitable dosage form with one or more other pharmaceutically acceptable excipients and optional

In one aspect, the present invention provides an immediate-release solid oral composition in the form of a tablet, wherein the tablet comprises one or more CFTR modulators such as Ivacaftor, Lumacaftor, Tezacaftor, and Elexacaftor, and one or more pharmaceutically acceptable excipients, wherein the disclosed composition is made by using the direct compression method and optionally coated with a film coating.

In another aspect, the present invention provides an immediate-release solid oral composition, which includes granules or pellets, wherein the granules or pellets comprise one or more CFTR modulators, such as Ivacaftor, Lumacaftor, Tezacaftor, and Elexacaftor, and one or more pharmaceutical acceptable excipients, wherein the disclosed composition is made by using any one of the granulation methods, such as wet granulation and dry granulation, or the direct compression method, wherein said composition can be processed into mini-tablets, tablets, or filled into capsules or unit dose packets.

In yet another aspect, the present invention provides an immediate-release solid oral composition in the form of a tablet or granules/pellets, wherein the tablet or granules/pellets comprises at least one CFTR potentiator, i.e., Ivacaftor or its salts or derivatives, wherein Ivacaftor is micronized and has a D₉₀ not more than 25 µm and D₅₀ not more than 15 µm, and wherein the disclosed composition is free of solid dispersion method, wherein said composition can be processed into mini-tablets, tablets, or filled into capsules or unit dose packets.

In yet another aspect, the present invention provides an immediate-release solid oral composition in the form of a tablet or granules/pellets, wherein the tablet or granules/pellets includes a). intra-granulation, b). extra granulation, and optionally c). film coating. Wherein Intra-granulation comprises micronized Ivacaftor and at least one binder, wherein granules are made by using wet granulation or dry granulation, wherein extra-granulation comprises at least one disintegrant and lubricant, and optionally coated with film coating.

In an aspect, the present invention provides a process of pharmaceutical composition comprising ivacaftor or a pharmaceutically acceptable salt thereof and one or more suitable pharmaceutically acceptable excipients in suitable amounts as described herein above in various aspects, wherein said process comprises preparing ivacaftor granules using any granulation method known in the art such as dry granulation methods, e.g. slugging or roller compaction, wet granulation methods, e.g., high-shear granulation (or rapid mixer granulation) or top spray granulation (using fluidized bed processor), or other granulation techniques such as twin screw granulation, etc., or one or more combinations thereof. In an embodiment, said process comprises a dry granulation technique. In one embodiment, said granules are prepared in the presence of a binder. In another embodiment, said granules are prepared in the absence of a binder.

In yet another aspect, the present invention provides an immediate-release composition that can be a solid oral composition, which includes pre-mixed micronized Ivacaftor with one or more pharmaceutically acceptable excipients, wherein the composition is made by using the direct compression method and wherein the said composition is lubricated with one or more lubrication agents and optionally coated with a film coating, wherein the disclosed composition can be processed into mini-tablets, tablets, or granules filled into capsules or unit dose packets.

In yet another aspect, the current innovators have developed a novel immediate-release composition that is thought to be extremely beneficial and free of solid dispersion. The current novel immediate-release compositions employ micronized ivacaftor and direct compression methods, which reduce manufacturing costs and other related issues; additionally, the use of micronized ivacaftor increases solubility.

### DETAILED DESCRIPTION OF THE INVENTION:

In one embodiment, the present invention provides novel solid oral pharmaceutical compositions comprising CFTR modulators, as well as a process for preparing them.

In another embodiment, the present invention provides novel solid oral compositions comprising cystic fibrosis transmembrane conductance regulator (CFTR) modulators such as CFTR potentiators and CFTR correctors. Ivacaftor is classified as a cystic fibrosis transmembrane conductance regulator (CFTR) potentiator and that is use to treat cystic fibrosis in patients who have a G551D mutation in the CFTR gene.

The active ingredients include CFTR potentiators, which are useful in the treatment of cystic fibrosis. The active ingredients include, but are not limited to, Ivacaftor, its salts, or derivatives. The disclosed formulations may also include one or more CFTR correctors, such as lumacaftor, tezacaftor, and elexacaftor.

In another embodiment, the present disclosure describes therapeutic compositions for the treatment of cystic fibrosis (CF) and other conditions or disorders that respond to the CFTR protein by providing dosage forms that deliver a therapeutic amount of active drug in an immediate release.

In another embodiment, ivacaftor is used in the disclosed formulations. The preferred pharmaceutically active substances are ivacaftor, lumacaftor, tezacaftor, and elexacaftor, as well as their active salts or derivatives. Ivacaftor (VX-770, N-(2,4-Di-tert-butyl-5-hydroxyphenyl)-4-oxo-1,4-dihydroquinoline-3-carboxamide) is a small molecule CFTR modulator that selectively targets the third most common genetic mutation in the CFTR gene. It restores the defective CFTR function by increasing the probability of CFTR channel opening at the cell surface. Vertex^{®} Pharmaceuticals developed the first CFTR modulator, Kalydeco^{®}, which was approved in 2012 in the form of an amorphous solid dispersion tablet.

Throughout this specification, the term "immediate release" is used in accordance with common understanding to refer to those dosage forms that allow drugs to be released directly in the gastrointestinal fluids, with no intention of delaying drug dissolution or absorption. According to the European Pharmacopoeia, in most cases the acceptance criteria for immediate-release dosage forms are that at least 80 percent of the active substance is released within a specified time, typically 45 minutes or less.

Throughout this specification, the term "micronized" refers to a micronized substance that has been reduced to a fine powder with particles measuring in microns. Ivacaftor is classified as a biopharmaceutical (BCS) Class 2 drug because of its high permeability and low solubility. In pharmaceutical preparations, particle size is the most important consideration. Achieving the ideal particle size can make materials easier to handle and process, improve the delivery of active pharmaceutical ingredients (APIs), and improve the quality or characteristics of raw materials. Because particle size is so important in pharmaceutical formulations, particle size reduction processes, such as micronization, are essential in the drug manufacturing process. Reducing a large, solid unit into fine particles measuring in the micrometer range may appear simple, but micronization technology involves a wide range of equipment and processes. Micronization refers to particle size reduction processes that produce particles smaller than 25 microns in diameter. In an ideal world, all oral formulation drugs would have high solubility and permeability, making it simple to create tablets or powders in capsules. But this is not always the case. Many small-molecule drugs have good permeability but poor solubility (low dissolution rate). Micronization is especially effective for these drugs, allowing formulations to completely dissolve in the small intestine.

In certain embodiments, the oral solid composition also includes one or more binders, such as cellulose derivatives (i.e., microcrystalline cellulose has versatile compressibility properties, making it highly useful in solid pharmaceutical dosage forms like tablets, acrylic acid derivatives, alginates, gelatin, polyvinylpyrrolidone, starch derivatives, dextrose, dextrates, dextrin, maltose, and maltodextrin; polyvinyl pyrrolidone (PVP) with a molecular weight ranging from about 4000 to about 1500000 and preferably about 30,000-1500000 can be used as a binder). Polyvinylpyrrolidone is available in different grades based on K-value and molecular weights, such as polyvinyl pyrrolidone with K values of 24-26, 29-32, or 85-95. Preferably polyvinyl pyrrolidone with K value 85-95 (Plasdone K-90/D^{®}, Kollidon 90F^{®}), hydroxypropyl methyl cellulose, hydroxypropyl cellulose, hydroxy ethyl cellulose, polyvinyl alcohol, sodium carboxy methyl cellulose, starches such as corn starch, modified corn starch, sugars, gum acacia, and the like.

In certain embodiments, a diluent or filler is also included in the oral dosage form. Diluents are fillers that are used to increase the bulk volume of a tablet or capsule. By combining a diluent with the active pharmaceutical ingredient, the final product is given adequate weight and size to assist in production and handling. Suitable examples of diluents to be used in accordance with the present invention include but are not limited to lactose, lactose monohydrate, spray- dried lactose monohydrate, anhydrous lactose, microcrystalline cellulose, pregelatinized starch, mannitol, corn starch, maltodextrin, dextrin, dextrose, erytritol, fructose, maltose, sucrose, xylitol, dicalcium phosphate, hydroxypropyl betadex, ammonium alginate, calcium carbonate, calcium phosphate, calcium phosphate dehydrate, neutral pellets, calcium sulfate, cellulose, cellulose acetate, dextrates, dextrin, ethylcellulose, glyceryl palmitostearate, magnesium carbonate, magnesium oxide, maltodextrin, medium chain triglycerides, polydextrose, polymethacrylates, sodium alginate, sodium chloride, sorbitol, starch, sugar sphericals, sulfobutylether beta-cyclodextrin, tragacanth, trehalose, polysorbate 80 or mixtures thereof.

In certain embodiments, one or more suitable disintegrants may be selected to be used in the invention, which include, but are not limited to, calcium carboxymethyl cellulose, crospovidone, cross-linked carboxymethyl cellulose sodium (croscarmellose sodium), starch, sodium carboxymethyl cellulose, carboxymethyl cellulose, docusate sodium, guar gum, polyacryline potassium, sodium alginate, sodium starch glycolate, alginic acid, alginates, ionexchange resins, magnesium aluminium silica, sodium dodesyl sulphate, poloxamer, sodium glycine carbonate, or mixtures thereof.

In one embodiment, a novel oral composition of the present invention can be administered as itself or formulated as an oral formulation (e.g., a solid dosage form such as tablets, mini-tablets, capsules (e.g., hard capsules and soft capsules), granules, and powders, or a liquid formulation such as a syrup, an emulsion, a suspension, and the like).

In another embodiment of a novel composition of the present disclosure is a dosage form that includes granules or pellets comprising one or more therapeutic agents, one or more binders, one or more fillers, and one or more disintegrants, wherein the compositions disclosed herein can be in the form of coated granules or pellets.

In another embodiment of a novel composition of the present disclosure is a dosage form that includes granules or pellets comprising ivacaftor and one or more pharmaceutically acceptable excipients, or the granules can consist essentially of the ivacaftor and one or more excipients coated on an inert pellet/bead. and optionally coated with a film coating, and the coated granules can be processed into mini-tablets, tablets, or filled into capsules or unit dose packets.

In yet another embodiment the present invention provides an immediate release solid oral composition in the form of tablet, which comprising one or more CFTR modulators such as CFTR potentiators and CFTR correctors, and one or more pharmaceutically acceptable excipients, wherein the disclosed composition is free of solid dispersion, wherein the tablet optionally coated with film coating solution.

In one embodiment, the present invention provides a novel immediate-release composition consisting granules containing micronized ivacaftor and one or more pharmaceutically acceptable excipients, wherein said granules are substantially free of solid dispersion method, wherein the composition made into a suitable dosage form with one or more excipients.

In one embodiment, the present invention provides a pharmaceutical solid oral composition comprising ivacaftor, wherein the ivacaftor is provided in a micronized amorphous form. The micronized amorphous ivacaftor is characterized by a high surface area and enhanced dissolution profile, which is incorporated into the solid oral dosage form with one or more pharmaceutically acceptable excipients. In a preferred aspect of this embodiment, the micronized amorphous ivacaftor maintains its amorphous state throughout the manufacturing process and within the final solid oral composition.

In one embodiment, the pharmaceutical solid oral composition of the present invention includes a micronized CFTR potentiator, such as ivacaftor. Ivacaftor has a D90 not more than 25 µm and D50 not more than 15 µm

In another embodiment, the present invention provides a solid oral immediate-release composition comprising a tablet or mini-tablet, which comprises micronized ivacaftor and one or more pharmaceutically acceptable excipients, wherein said composition is made by using the direct compression method, wherein tablets or mini-tablets are optionally coated with a film coating, and wherein mini-tablets can be supplied as a co-packaged blister pack with an additional one or more therapeutic agents.

In a preferred embodiment, the invention provides to a solid oral dosage form, comprising a therapeutically effective amount of micronized ivacaftor. The micronized ivacaftor exhibits a specific surface area and particle size profile optimized for rapid dissolution, wherein, D₉₀ particle size of not more than 25 µm, preferably between 25 µm and 15 µm; and D₅₀ particle size of not more than 15 µm, preferably between 5 µm and 15 µm.

In one embodiment, the present invention provides a pharmaceutical solid oral dosage form in the form of a tablet comprising micronized amorphous ivacaftor and at least one pharmaceutically acceptable excipient. The micronized amorphous ivacaftor is characterized by a controlled particle size distribution (PSD) configured to enhance the dissolution rate and oral bioavailability of the active agent.

Specifically, the micronized amorphous ivacaftor possesses a D₉₀ particle size of not more than 25 µm, preferably between 25 µm and 15 µm; and D₅₀ particle size of not more than 15 µm, preferably between 5 µm and 15 µm.

The tablet may be manufactured by processes known in the art, including direct compression or dry granulation. The at least one pharmaceutically acceptable excipient is selected from the group consisting of fillers, surfactants, disintegrants, binders, and lubricants.

In another embodiment, the present invention provides a pharmaceutical composition in a solid oral dosage form selected from the group consisting of minitablets and granules. The composition comprises micronized amorphous ivacaftor characterized by an ultra-fine particle size distribution (PSD).

Specifically, the micronized ivacaftor for granules according to this embodiment is characterized by a D₉₀ particle size of about 5 µm, a D₅₀ particle size of about 2.5 µm, and a D₁₀ particle size of about 1 µm.

In another embodiment, the present invention provides a solid oral immediate-release composition comprising ivacaftor and a method of preparation, wherein the method of preparation includes a) pre-mixing, which includes micronized ivacaftor and at least one pharmaceutically acceptable excipient, b). blending, which includes pre-mixed ivacaftor and other pharmaceutically acceptable excipients, and c). lubrication, which includes blended ivacaftor granules and at least one or more lubricating agents, wherein the disclosed composition is prepared into a suitable dosage form with one or more pharmaceutically acceptable excipients.

In another embodiment, the present invention provides a solid oral immediate-release composition in the form of granules, wherein the granules comprise micronized ivacaftor having D90 not more than 25 µm and D50 not more than 15 µm, and a method of preparation, which includes a) pre-mixing the micronized ivacaftor with at least one pharmaceutically acceptable excipient, preferably a binder or diluent. b) blending the pre-mixed ivacaftor powder with one or more other pharmaceutically acceptable excipients, and c) lubricating the blended Ivacaftor granules with one or more lubricating agents, where preferable lubricating agents include but are not limited to magnesium stearate, stearic acid, calcium stearate, and sodium stearyl fumarate, wherein the disclosed composition is prepared into a suitable dosage form with one or more pharmaceutically acceptable excipients.

In one embodiment, the present invention provides to a novel pharmaceutical composition comprising Ivacaftor or a pharmaceutically acceptable salt thereof and one or more pharmaceutically acceptable excipients, wherein said composition comprises an intra-granular portion and an extra-granular portion.

In another aspect, the present invention provides a pharmaceutical composition comprising:
a) intragranular portion comprising: about 20% to 40% w/w, preferably about 25% to 35% w/w of Micronized Ivacaftor; about 20% to 80% w/w, preferably about 30 to 70% w/w of one or more diluents; about 2% to 30% w/w, preferably about 3 to 20% w/w of one or more disintegrants; about 0.1 to 10% w/w, preferably about 0.3 to 3% w/w of one or more surfactants, based on the total weight of the composition; and
b) extra granular portion comprising: about 0.1 to 10% w/w, preferably about 0.5 to 5% w/w of one or more lubricants, and optionally about 0.5% to 10 % w/w of one or more disintegrants; based on the total weight of the composition;
In one embodiment said composition may further comprise a binder, a glidant and/or other suitable excipients in suitable amounts as understood by a skilled person in the art.

In yet another embodiment, the present invention provides a solid oral immediate-release composition in the form of granules or tablets and a method of their preparation, wherein the method of preparation includes:
I) Intra-granulation process: a) pre-mixing micronized ivacaftor with at least one pharmaceutically acceptable excipient. b) granulating the pre-mixed powder with a binder solution to form a granule, where the binder solution consists of a binding agent and a solvent, and preferable binders include but are not limited to cellulose, methyl cellulose, polyvinyl pyrrolidine, PEG solution binders, gelatine, PVP, HPMC, PEG, sucrose, and starch. c) dry the granules at an appropriate temperature.
II) Extra granulation process: a) blending the dried drug granules with one or more other extra granular excipients, which preferably include a disintegrant and a filler, and b) lubricating the blended Ivacaftor granules with one or more lubricating agents, which include but are not limited to magnesium stearate, stearic acid, calcium stearate, and sodium stearyl fumarate. c) compressing lubricated granules into tablets, mini-tablets, or granules packed into capsules or unit dose packets.

In yet another embodiment, the immediate-release composition of the present invention provides a composition in the form of a tablet and its method for preparing it, wherein the composition is made by using any one of the granulation methods, such as wet granulation and dry granulation, or the direct compression method. The present invention preferably used direct compression method, wherein the said method of preparing a tablet comprises followed steps:
a) pre-mixing the micronized Ivacaftor with a binder or a filler;
b) blending the pre-mixed Ivacaftor powder with one or more pharmaceutically acceptable excipients, along with an additional therapeutic agent, preferably lumacaftor;
c) lubricating the blended powder with at least one lubricating agent;
d) compressed the lubricated powder into tablets or mini-tablets, with optimal hardness; and
e) optionally coated the tablets with film coating.

In yet another embodiment, the immediate release composition of the present invention provides a composition in the form of granules and its process for the preparation, wherein the disclosed composition is free of solid dispersion method, wherein the granules are made by using the direct compression method, wherein said process for preparing the granules comprises the following steps:
a) pre-mixing the micronized Ivacaftor with at least one binder or a diluent.
b) blending the pre-mixed Ivacaftor powder with one or more pharmaceutically acceptable excipients along with one or more additional therapeutic agents, which can be CFTR correctors such as lumacaftor, Tezacaftor, and Elexacaftor, preferably Tezacaftor.
c) lubricating the blended powder with at least one lubricating agent, and
d) optionally coating the granules with a film coating solution, wherein the disclosed composition is prepared into a suitable dosage form, it can be processed into tablets, mini-tablets, or filled into capsules or unit dose packets.

In yet another embodiment, the immediate-release solid oral composition of the present invention provides a composition in the form of tablets or mini-tablets and processes for their preparation, wherein the composition is made by using the direct compression method, wherein said granules comprise micronized Ivacaftor, wherein the disclosed composition is free of the solid dispersion method, and wherein said process for preparing the tablets comprises the following steps:
a) pre-mixing the Ivacaftor with at least one binder or a diluent.
b) blending the pre-mixed Ivacaftor powder with one or more pharmaceutically acceptable excipients along with one or more additional therapeutic agents, which can be CFTR correctors such as lumacaftor, tejacaftor, and elexacaftor, preferably tezacaftor and elexacaftor.
c) lubricating the blended powder with one or more lubricating agents.
d) compress the lubricated powder into a tablet or mini-tablet with optimal hardness, and
e) optionally coat the tablets or mini-tablets with a film coating solution, wherein mini-tablets can be supplied as a co-packaged blister pack with an additional Ivacaftor mini-tablet.
further embodiment, the present invention provides a solid oral immediate release granules composition and method of their preparation, wherein granules comprise Micronized Ivacaftor and one or more pharmaceutically acceptable excipients, wherein said method of preparing granules composition uses direct compression method, wherein the method which includes micronized Ivacaftor admixed with at least one binder or a diluent, blending the ivacaftor admixture with at least one disintegrant and surfactant, wherein preferable disintegrant includes but are not limited to croscarmellose sodium, crospovidone, and sodium starch glycolate, lubricating the blended ivacaftor mixture with at least one lubricating agent, wherein preferable locating agents includes but are not limited to magnesium stearate and Silicone dioxide (also known as colloidal silicon dioxide), wherein the granules can be processed into tablets or mini-tablets that are optionally coated with film coating, wherein mini-tablets can be supplied as co-packaged blister pack with an additional one or more therapeutic agents.

In one embodiment, the present invention provides a novel pharmaceutical solid oral composition comprising Ivacaftor, one or more carriers / diluents selected from the group include, but are not limited to lactose, Mannitol, starch, cellulose, microcrystalline cellulose, and cellulose derivatives, e.g. methylcellulose, and the like, one or more disintegrants selected from the group include, but are not limited to croscarmellose sodium, sodium starch glycolate, pregelatinized starch, cross-linked polyvinylpyrrolidone and the like, one or more surfactant selected from the group include, but are not limited to ammonium lauryl sulfate, sodium lauryl sarcosinate, sodium pareth sulfate, sodium stearate, sodium lauryl sulfate, α olefin sulfonate, and ammonium laureth sulfate.

In another embodiment, the compositions of the present disclosure can also include one or more functional excipients, such as lubricants, anti-tacking agents, anti-static agents, thermal lubricants, antioxidants, buffering agents, alkalinizing agents, binders, diluents, sweeteners, chelating agents, colorants, flavorants, surfactants, solubilizers, wetting agents, stabilizers, hydrophilic polymers, hydrophobic polymers, waxes, lipophilic materials, absorption enhancers, preservatives, absorbents, cross-linking agents, bioadhesive polymers, retardants, pore formers, and fragrance.

Examples of these pharmaceutically acceptable excipients in the component of the current invention include, but are not limited to, lubricants or anti-tacking agents: talc, fumed silicon dioxide, colloidal silica, titanium dioxide, kaolin, magnesium stearate, calcium stearate, stearic acid, and hydrogenated vegetable oils; surfactants: polysorbate 80, sorbitan monooleate, polyoxymer, and sodium lauryl sulfate.

### EXAMPLES:

The invention is further illustrated by the following examples, which are provided to be exemplary of the invention and do not limit the scope of the invention. While the present invention has been described in terms of its specific embodiments, certain modifications and equivalents will be apparent to those skilled in the art and are intended to be included within the scope of the present invention.

The active ingredient in the example is not limited and can be substituted with other active salts of CFTR modulators or combinations thereof.

The Particle Size Distribution (PSD) values of Ivacaftor API are measured by means of a laser diffraction particle size analyser (e.g. Malvern Mastersizer 2000) Sample handling unit : Hydro2000S (A). An API sample is mixed with a liquid dispersant (paraffin liquid light) and sonicated with continuous stirring. The sample solution is introduced in the sample handling unit of the above-mentioned instrument, in which a laser beam is passed through the sample. Sample particles scatter the light at different angles depending on their size. The instrument detectors measure the intensity of the scattered light at different angles, and the software applies optical models to convert the scattering pattern into a particle size distribution curve. The mean particle size values are extracted from the distribution curve. These are statistical parameters used to describe the average particle size and the distribution of particles in a sample. In this analysis several mean values (such as the below) are used because particles are not all the same size.
- D10 (Lower Percentile Size) is the particle diameter at which 10% of the total particles are smaller than this size. It indicates the fine fraction of the sample and is useful for understanding how many very small particles are present.
- D50 (Median Particle Size or Median Diameter) is the particle diameter where 50% of particles are smaller and 50% are larger. It is the most commonly used average particle size value.
- D90 (Upper Percentile Size) is the particle diameter at which 90% of particles are smaller than this size. It represents the coarse fraction of the sample.

### Example 1:

An immediate-release composition comprising Ivacaftor was prepared by the direct compression method and has the composition as given in Table 1.

**Table 1:**

| **Sr. No** | **component** | **Percentage w/w** |
|---|---|---|
| 1 | Micronized Ivacaftor D90 not more than 25 µm and D50 not more than 15 µm | 25 - 40 |
| 2 | Diluent / filler | 25 - 40 |
| 3 | Binder | 25 - 40 |
| 4 | Disintegrant | 1 - 10 |
| 6 | Lubricant | 0.2 - 1 |

An Immediate release Ivacaftor formulations were prepared by the direct compression method, which includes dispensing 25-40% of micronized ivacaftor and a diluent and sieve for deagglomeration, pre-mix the sieved micronized ivacaftor with diluent, dispensing the required amount of binding agent and disintegrating agent, and sieve for deagglomeration; blending the ivacaftor pre-mixture with binding agent, and disintegrating agent was placed in a container and mix for around 10 minutes to obtain a blend and dispensing the required amount of lubricating agent and then sieve for deagglomeration; and lubricating the blend with magnesium stearate to obtain a homogeneous blend. Finally, collect the lubricated, homogeneous blend and compress it into mini-tablets, tablets, or granules that can be placed into capsules or unit dose packs. prepared tablets or mini-tablets optionally coated with film coating.

### Example 2:

The solid oral composition comprising Ivacaftor were prepared by wet granulation method and have the composition as given in table 2.

**Table 2:**

| **Sr. No** | **Component** | **Percentage w/w** |
|---|---|---|
| **Intra granular portion** | | |
| 1 | Micronized D90 not more than 25 µm and D50 not more than 15 µm | 25 - 40 |
| 2 | Lactose monohydrate | 15 - 30 |
| 3 | Micro crystalline cellulose (MCC) | 25 - 40 |
| 4 | water | qs |

| **Extra granular portion** | | |
|---|---|---|
| 5 | Lactose monohydrate | 10 - 20 |
| 6 | Sodium lauryl sulphate (SLS) | 0.2 - 0.8 |
| 7 | Croscarmellose sodium | 1 - 10 |
| 8 | Silicon dioxide | 0.5 - 1 |
| 9 | Magnesium stearate | 0.5 - 1.5 |

The solid oral ivacaftor compositions were prepared by using the wet granulation method, which includes dispensing 25-40% of micronized ivacaftor and 20-30% of lactose monohydrate and sieving for deagglomeration, and a granulating solution was prepared by mixing microcrystalline cellulose and water in a container. Load the sieved ivacaftor and lactose into the rapid mixture granulator and pour the binder solution in an appropriate time to obtain granules; collect and dry the wet granules and sieve for deagglomeration; dispense and sieve all extra granular excipients; and blend the dried granules with all extra granular excipients other than magnesium stearate in a blender to obtain a homogeneous blend; lubricate the homogeneous blend with sieved magnesium stearate; and collect the lubricated homogeneous blend and compress it into mini-tablets or tablets or fill it into capsules or unit dose packets. prepared tablets or mini-tablets optionally coated with film coating.

### Example 3:

The solid oral immediate release composition comprising Ivacaftor were prepared by dry granulation method and have the composition as given in table 3.

**Table 3:**

| **Sr. No** | **Component** | **Percentage w/w** |
|---|---|---|
| **Intra granular portion** | | |
| 1 | Micronized Ivacaftor D90 not more than 25 µm and D50 not more than 15 µm | 25 - 40 |
| 2 | Lactose monohydrate | 15 - 30 |
| 3 | Micro crystalline cellulose (MCC) | 25 - 40 |
| 4 | Croscarmellose sodium | 1 - 10 |
| 5 | Sodium lauryl sulphate (SLS) | 0.1 - 0.5 |
| 6 | Silicon dioxide | 0.2 - 0.8 |
| 7 | Magnesium stearate | 0.2 - 0.8 |

| **Extra granular portion** | | |
|---|---|---|
| 8 | Lactose monohydrate | 10 - 20 |
| 9 | Sodium lauryl sulphate (SLS) | 0.1 - 0.5 |
| 10 | Croscarmellose sodium | 1 - 5 |
| 11 | Silicon dioxide | 0.1 - 0.5 |
| 12 | Magnesium stearate | 0.2 - 0.8 |

The solid oral Immediate Release Ivacaftor compositions were made using the dry granulation method, which involved dispensing 25-40% of micronized ivacaftor, 20-30% of lactose monohydrate, 15-30% of MCC, 1-5% of SLS, and 0.2-0.8% of silicon dioxide and sieving for deagglomeration. After that, the components were placed in a container and blended for 10 minutes with a diffusion blender to obtain a blend; 0.2-0.8% magnesium stearate was dispensed and sieved for deagglomeration. The blend was then homogenized by mixing it with magnesium stearate for three minutes with a diffusion blender to obtain a homogeneous blend. Slugs were formed by compressing the blend. The slugs were then crushed and sieved to obtain uniform granules.
dispensed 25-40%of micronized ivacaftor, 20-30%of lactose monohydrate, 15-30%of MCC, 1-5%of SLS, 0.2-0.8% of silicon dioxide were weighed and sieved for deagglomeration. Afterwards, the components were placed in a container together with the granules. and mixed for 10 minutes using a diffusion blender obtaining a blend, 0.2-0.8% of magnesium stearate was dispensed and sieved for deagglomeration. Then the blend was mixed with the magnesium stearate for 3 minutes using a diffusion blender to obtain a homogeneous blend. Finally, this blend and compressed into mini-tablets or tablets, or filled into capsules or unit dose packets. prepared tablets or mini-tablets optionally coated with film coating.

### Example 4:

The solid oral an immediate release pharmaceutical composition comprising Ivacaftor were prepared by direct compression method and have the composition as given in table 4.

**Table 4:**

| **Sr. No** | **Component** | **Percentage w/w** |
|---|---|---|
| Core composition | | |
| 1 | Micronized Ivacaftor D90 not more than 25 µm and D50 not more than 15 µm | 25-40 |
| 2 | Lactose monohydrate | 30 - 34 |
| 3 | Micro crystalline cellulose (MCC) | 30 - 34 |
| 4 | Croscarmellose sodium | 2-8 |
| 5 | Sodium lauryl sulphate (SLS) | 0.2 - 0.4 |
| 6 | Colloidal Silicon dioxide (Aerosil 200) | 0.5 |
| 7 | Magnesium stearate | 0.5 - 1 |
| Optional film coating | | |

The solid oral immediate-release ivacaftor composition were prepared by using the direct compression method, which includes dispensing 25-40% of micronized ivacaftor, 30-34% of lactose monohydrate, 30-34% of microcrystalline cellulose (MCC), 2-8% of Croscarmellose sodium, 0.2-0.4% of sodium lauryl sulphate (SLS), 0.5% of colloidal silicon dioxide (Aerosil 200), and sieving it for deagglomeration, and were mixed in a blender for around 5-15 minutes to obtain a blend, dispensing 0.5-1% of magnesium stearate, sieving it for deagglomeration, and adding it into the obtained blend and continuing mixing for around 5 to 10 minutes to obtain a homogeneous blend, collect the final lubricated homogeneous blend and compress it into mini-tablets or tablets, or fill it into capsules or unit dose packs. Film coating is optional for produced tablets or mini-tablets.

### Example 5:

Pharmaceutical Tablet Compositions Comprising Ivacaftor and Dry Granulation Methods for Manufacturing the Same

| **Sr. No** | **Component** | **Quantity per tablet** |
|---|---|---|
| Core tablet | | |
| 1 | Ivacaftor micronized (D90: 15-25 µm; D50: 5-15 µm). | 150 mg |
| 2 | Lactose monohydrate DC | 184.59 mg |
| 3 | Microcrystalline cellulose | 182.7 mg |
| 4 | Croscarmellose sodium | 16.2 mg |
| 5 | Sodium lauryl sulfate | 1.77 mg |
| 6 | Colloidal silicon dioxide | 2.7 mg |
| 7 | Magnesium stearate | 5.04 mg |

| Film Coating | | |
|---|---|---|
| 8 | Opadry II blue | 12 mg |
| 9 | Purified water | QS |

### A Process for Preparing a Stable Ivacaftor Tablet via Dry Granulation

### Step A: Intragranular Blending and Dry Granulation

1. **Sifting:** Co-sift Lactose Monohydrate and Microcrystalline Cellulose through a 500 µm aperture screen using a vibratory Russell Finex sifter.
2. **Primary Blend:** Charge the sifted filler-binders into a suitable Double Cone Blender and add the Ivacaftor drug substance.
3. **Premix:** Prepare a geometric dilution of Sodium Lauryl Sulfate and approximately two-thirds of the total required Croscarmellose Sodium in a closed environment. Add this premix to the Double Cone Blender.
4. **Dry-Blending:** Blend the composite mixture for a period of 10 to 15 minutes to ensure active ingredient uniformity.
5. **Pre-Slugging Lubrication:** Sift Colloidal Silicon Dioxide and 50% of the total Magnesium Stearate through a 250 µm screen. Add to the blender and lubricate for 1 to 2 minutes.
6. **Compaction (Slugging):** Compress the lubricated blend into slugs using a heavy-duty tablet press equipped with 15 mm round tooling.
7. **Milling:** Mill the resulting slugs through a 2 mm screen using a FitzMill to produce the intragranular granules.

### Step B: Extra granular Blending and Compression

**8. Extra granular Blending:** Charge the milled granules into a Double Cone Blender. Add the remaining one-third of the Croscarmellose Sodium and blend for 2 to 3 min.
**9. Final Lubrication:** Sift the remaining 50% of the Magnesium Stearate through a 250 µm screen, add to the mixture, and perform final lubrication for 1 to 2 minutes.
**10. Tablet Compression:** Compress the final blend into core tablets using 16.2 x 8.2 mm oblong tooling. The target hardness is 8 to 10 Kp.

### Step C: Film Coating

**11. Coating Suspension:** Prepare a 20% w/w solids aqueous dispersion of Opadry II Blue. Maintain a vortex during the 5-minute addition phase and continue mechanical stirring for 20 to 30 minutes to ensure complete polymer hydration.
**12. Coating:** Charge the core tablets into a perforated coating pan. Pre-warm the tablet bed to an equilibrium temperature of 55°C. Begin the spray cycle and continue until the coated tablet weight reaches a target weight gain.

### Example 6:

Ivacaftor Mini-Tablet / Granule Compositions and Methods for the Manufacture Thereof.

| **Sr. No** | **Component** | **Quantity per minitablet** |
|---|---|---|
| Intra granular | | |
| 1 | Ivacaftor micronized (D90 not more than 5 µm, D50 not more than 2.5 µm) | 1.923 mg |
| 2 | Lactose monohydrate DC | 1.499 mg |
| 3 | Mannitol | 1.555 mg |
| 4 | Croscarmellose sodium | 0.9 mg |
| 5 | Sodium lauryl sulfate | 0.134 mg |
| 6 | Colloidal silicon dioxide | 0.035 mg |
| 7 | Magnesium stearate | 0.101 mg |
| 8 | Sucralose | 0.092 mg |

| Extra granular | | |
|---|---|---|
| 9 | Croscarmellose sodium | 0.44 mg |
| 10 | Colloidal silicon dioxide | 0.0165 mg |

### Manufacturing Process for Ivacaftor Mini-Tablets

### Stage 1: Intragranular Blending and Dry Granulation

1. **Sifting:** Co-sift Lactose Monohydrate and Mannitol through a 500 µm aperture screen using a vibratory sifter (e.g., a Russell Finex sifter).
2. **Primary Blend:** Charge the sifted fillers into a Double Cone Mixer and add a therapeutically effective amount of Ivacaftor.
3. **Premixing:** Prepare a geometric dilution of Sodium Lauryl Sulfate and the intra granular portion of Croscarmellose Sodium in a separate container. Add this premix to the Double Cone Mixer.
4. **Homogenization:** Blend the composite mixture for a period of 10 to 15 minutes to ensure uniform drug distribution.
5. **Pre-Compaction Lubrication:** Sift the intragranular portion of Colloidal Silicon Dioxide and Magnesium Stearate through a 250 µm screen. Add to the blender and lubricate for 1 to 2 minutes.
6. **Compaction (Slugging):** Subject the lubricated blend to dry granulation, either by slugging using 13 mm round punches or by roller compaction using a Chilsonator to form a compacted mass.
7. **Comminution (Milling):** Mill the resulting slugs or ribbons through a 0.4 mm screen using a FitzMill to achieve a target granule size distribution suitable for mini-tablet compression.

### Stage 2: Extragranular Blending and Mini-Tablet Compression

8. **Extragranular Integration:** Charge the milled granules into a Double Cone Mixer. Add two-thirds of the extragranular portion of Croscarmellose Sodium and blend for approximately 5 minutes.
9. **Final Lubrication:** Sift the Colloidal silicon dioxide through a 250 µm screen, add to the mixer, and perform final lubrication for 1 to 2 minutes.
10. **Mini-Tablet Compression:** Compress the final blend into a plurality of mini-tablets using a tablet press equipped with multiple-tip Type-D, 2 mm diameter, round flat-faced punches. The mini-tablets are compressed with a target hardness of 1 Kp (around 10 N).

## Claims

1. A pharmaceutical solid oral composition comprising micronized ivacaftor or a pharmaceutically acceptable salt thereof, and at least one pharmaceutically acceptable excipient.

2. The pharmaceutical composition of claim 1, wherein the composition is in the form of a tablet, and wherein the micronized ivacaftor has: (a) D₉₀ particle size of not more than 25 µm, preferably between 25 µm and 15 µm; and D₅₀ particle size of not more than 15 µm, preferably between 5 µm and 15 µm.

3. The pharmaceutical composition of claim 1, wherein the composition is in a form selected from minitablets and granules, and wherein the micronized ivacaftor has: (a) D₉₀ particle size of about 5 µm; (b) D₅₀ particle size of about 2.5 µm; and (c) D₁₀ particle size of about 1 µm.

4. The pharmaceutical composition of claim 1, wherein the composition is in a form selected from the group consisting of a tablet, a plurality of mini-tablets, granules, and a capsule; and wherein the composition is prepared by direct compression or dry granulation.

5. The pharmaceutical composition of any preceding claim, wherein the composition comprises a physical mixture of the micronized ivacaftor and the at least one pharmaceutically acceptable excipient, wherein the composition is a direct compression dosage form, and further wherein the composition is substantially free of a polymer-based solid dispersion.

6. A process for the preparation of a pharmaceutical solid oral immediate-release composition in the form of mini-tablets or granules, comprising the steps of dry granulating a primary blend comprising a therapeutically effective amount of ivacaftor, or a pharmaceutically acceptable salt thereof, and at least one pharmaceutically acceptable excipient to form granules; and compressing said granules into a plurality of mini-tablets; wherein the process is performed without the use of a liquid solvent and the resulting mini-tablets are substantially free of a solid dispersion.

7. The process of claim 7, further comprising mixing the granules with at least one extra granular excipient prior to the compressing step, wherein the extra granular excipient is selected from a disintegrant, a lubricant, or a combination thereof.

8. A process for the preparation of a pharmaceutical solid oral immediate-release composition comprising ivacaftor, or a pharmaceutically acceptable salt thereof, the process comprising the steps of:
(a) dry granulating a primary blend comprising ivacaftor and at least one intragranular excipient to form granules;
(b) mixing said granules with at least one extra granular excipient to form a final blend;
(c) compressing the final blend into core tablets; and
(d) coating the core tablets with a film-coating suspension;
wherein the process is performed without a liquid solvent during the granulation steps, and the resulting composition is substantially free of a solid dispersion.

9. The process of claim 9, wherein the ivacaftor is in a micronized amorphous form prior to the dry granulating step, and wherein the process further comprises compressing the final blend into tablets having a hardness of about 8 kP to about 10 kP.

10. The pharmaceutical solid oral composition of any one of the preceding claims, wherein the ivacaftor is in a micronized amorphous form.
